# EUROPEAN PATENT APPLICATION

(11) **EP 3 591 066 A1**
(43) Date of publication of application: **08.01.2020**
(21) Application number: 18182042.4
(22) Date of filing: 05.07.2018
(51) Int. Cl.: C12Q 1/34

(54) **METHOD FOR DETECTING SYNOVIAL JOINT INFECTIONS**

(71) Applicant: Qualizyme Diagnostics GmbH&Co KG, 8010 Graz (AT)
(72) Inventor: Heinzle, Andrea, 8010 Graz (AT); Sigl, Eva, 8010 Graz (AT); Luschnig, Daniel, 8010 Graz (AT); Gamerith, Clemens, 8010 Graz (AT)
(74) Representative: Pföstl, Andreas

(57) **Abstract**

The present invention relates to a method for detecting a synovial joint infection comprising the steps of
- contacting a synovial fluid sample with at least one lysozyme substrate, and
- detecting a synovial joint infection when a conversion of said at least one substrate is determined

## Description

### TECHNICAL FIELD

The present invention relates to methods for detecting infections in synovial joints.

### BACKGROUND ART

The infection of joints such as knees, hips or shoulders is usually the result of a surgical procedure or injuries in which bacteria or viruses can enter the joints. Thus, artificial joints or implants are often affected by such infections. The detection of a bacterial infection, for instance, in joints is carried out by means of a joint puncture with synovial analysis, whereby the bacteria are detected by culturing on special culture media in an incubator. However, a negative culture result does not prove that it is free of any germs, as there are up to 30% false negative tests (Simank HG et al. (2004) Joint Anemia Orthopedist 33: 327-31). This microbial cultivation detection system usually takes 2 to 5 days. Alternatively, a leukocyte count can be performed in the synovial fluid (leukocyte / mm3). However, leukocyte counts between 1,000 and 10,000 do not preclude bacterial infection (Carpenter CRet al. (2011) Acad Emerg Med 18: 781-96; Coutlakis PJet al. (2002) J Clin Rheumatol 8: 67-71).

Septic arthritis, also known as infectious or - if bacteria are involved - bacterial arthritis is a dangerous disease. Depending on the germ, it can lead to considerable damage in the joint within a very short time. This is especially true for infection with *Staphylococcus aureus.* In addition, it can lead to a seeding of the pathogens into the bloodstream and, as a consequence, to a life-threatening general infection (sepsis).

In the case of bacterial arthritis, the fastest possible diagnosis and immediate therapy is absolutely critical to the outcome of the disease and the issue of complete healing. Every day without diagnoses, the bacteria in the joint cause damage that can never be fully repaired. In addition, each day of untreated infection increases the risk of spreading the pathogens to other parts of the body and thus the risk of life-threatening illness.

In the case of bacterial infections, treatment with antibiotics is necessary, implants must be removed most of the time. On the other hand, there are often inflammatory processes in the various joints, which also lead to swelling, redness and pain. In contrast to bacterial infections, the administration of antibiotics is not necessary in these cases. Instead, anti-inflammatory therapy will be administered. For accurate diagnosis, the affected joint therefore needs to be punctured.

Methods known in the art cannot be used to distinguish a synovial joint infection from a non-infectious inflammatory condition of a synovial joint in a reliable manner. However, such a distinction is of major importance because it allows to determine how a synovial joint disorder can be efficiently treated. If the cause of the disorder is a bacterial infection, for instance, antibiotics have to be administered to the mammal suffering from a synovial joint disorder.

Hence, it is an object of the present invention to provide a method for detecting synovial joint infections within minutes and to discriminate between synovial joint disorders caused by bacteria, for instance, and non-infectious inflammatory conditions.

### SUMMARY OF THE INVENTION

The present invention relates to a method for detecting a synovial joint infection comprising the steps of
- contacting a synovial fluid sample with at least one lysozyme substrate, and
- detecting a synovial joint infection when a conversion of said at least one substrate is determined.

It turned surprisingly out that a synovial joint infection can be detected by determining lysozyme activity in the synovial fluid of a mammal suffering from a synovial joint condition, wherein the enzymatic activity is preferably determined in an untreated synovial fluid sample obtained directly from said mammal (i.e. obtained by puncturing a synovial joint of a mammal). Until now it was thought that all kind of synovial joint conditions - either caused by infections or by non-infectious inflammation - result in an increase of lysozyme activity. However, as herein shown lysozyme activity in synovial fluid from mammals suffering from a non-infectious inflammatory condition of a synovial joint do not or not significantly show lysozyme activity. This finding is even more surprising considering that the activity of myeloperoxidase - a well known marker for infectious conditions - shows also activity in infected synovial fluid whereas elastase and Cathepsin G, for instance, - two further known markers for infectious conditions - do not show any enzymatic activity. These findings are very important because they allow to unequivocally identify synovial joint infections in mammals known to suffer from a synovial joint condition. The identification of the cause of the synovial joint conditions makes it possible to choose the right treatment for said condition. For instance, a bacterial infection of a synovial joint can be treated using antibiotics whereas a non-infectious inflammatory condition requires other treatment steps.

The findings of the present invention allow to differentiate synovial joint conditions into synovial joint infection and a non-infectious inflammatory condition within minutes.

Thus, another aspect of the present invention relates to a method for discriminating between a synovial joint infection and a non-infectious inflammatory condition of a synovial joint comprising the steps of
- contacting a synovial fluid sample of a mammal showing symptoms of a synovial joint condition with at least one lysozyme substrate,
- detecting a synovial joint infection when a conversion of said at least one substrate is determined in said sample and
- detecting a non-infectious inflammatory condition when substantially no lysozyme activity is determined.

The present invention describes an *in vitro* system for detecting infections, in particular bacterial infections, in mammalian synovial fluids. Infections can be identified by the method of the present invention within minutes since the lysozyme activity in infectious joints is significantly higher compared to healthy mammals and mammals suffering from non-infectious inflammatory condition.

In a preferred embodiment of the present invention substrates are used for the conversion with lysozyme which are able to release a marker which may be detected optically using, for instance, photometric methods or test stripes. Such a test system represents a tremendous decision-making aid for further treatment, since it can distinguish between bacterial and inflammatory processes in the joints of mammals, in particular humans.

The present invention enables a rapid result in terms of a possible infection directly in the ambulance or in the case of horses and cattle in the barn. The result is immediate, no lab or time-consuming microbiology is needed for the first diagnosis. Enzymes in synovial fluids, which are secreted by the body's immune system in case of bacterial joint infections to an increased extent, are detected preferably by a simple and rapid color reaction system. The targeted preparation of the sample material enables the sample to be quickly and reliably transferred to the new test system. Experimental data clearly show that in the case of a synovial infection different enzymes are significantly increased and a distinction between bacterial and inflammatory events is possible.

### BRIEF DESCRIPTION OF THE FIGURES

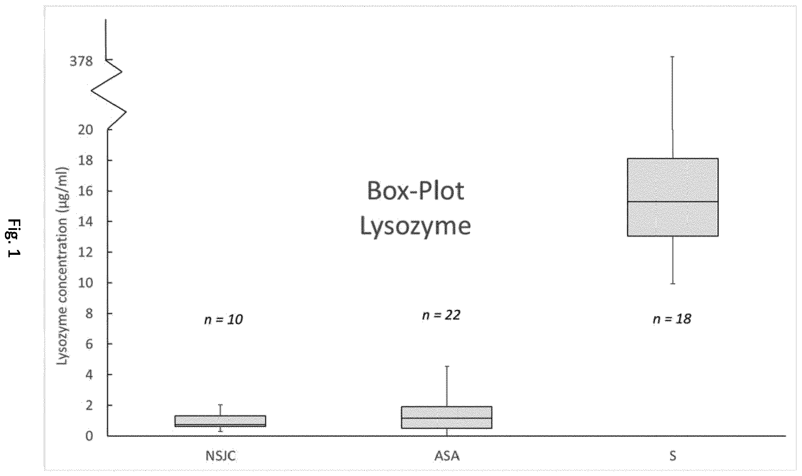

Fig. 1 shows the Box-Plot of Lysozyme concentrations in no synovial joint condition (NSJC), aseptic arthritic (ASA) and septic (S) synovia samples. A total amount of 50 samples was analysed, where 10 samples had no synovial joint conditions, 22 were aseptic arthritic and 18 samples were septic. The highest values of the different groups were as follows: NSJC 2.0 µg/ml, ASA 4.5 µg/ml, S 377.7 µg/ml. The median of the different groups were: NSJC 0.74 µg/ml, ASA 1.2 µg/ml and S 15.3 µg/ml. The lowest values for the three different groups were: NSJC 0.30 µg/ml, ASA 0.0 µg/ml and S 9.9 µg/ml.
Fig. 2 shows the Box-Plot of MPO concentrations in no synovial joint condition (NSJC), aseptic arthritic (ASA) and septic (S) synovia samples. A total amount of 50 samples was analysed, where 10 samples had no synovial joint conditions, 22 were aseptic arthritic and 18 samples were septic. The highest values of the different groups were as follows: NSJC 0.054 µg/ml, ASA 0.41 µg/ml, S 58.6 µg/ml. The median of the different groups were: NSJC 0.046 µg/ml, ASA 0.046 µg/ml and S 1.1 µg/ml. The lowest values for the three different groups were: NSJC 0.046 µg/ml, ASA 0.046 µg/ml and S 0.046 µg/ml.
Fig. 3 shows the absorbance values measured in the lysozyme activity assays according to example 2.
Fig. 4 shows the time-depending dye release from peptidoglycan-reactive black 5 using increasing concentrations of lysozyme.
Fig. 5 shows the influence of the substrate concentration on the reaction kinetics of lysozyme.

### DESCRIPTION OF EMBODIMENTS

The present invention relates to a method for detecting a synovial joint infection.

It turned surprisingly out that lysozyme activity in synovial fluid is a marker for infections in synovial joints since synovial fluid obtained from healthy joints as well as from joints from mammals suffering a non-infectious inflammatory condition of a synovial fluid (e.g. arthritis, in particular rheumatoid arthritis). This is particularly surprising because according to Pruzanski W et al. (Arth Rheumat (1970) 13:389-399), for instance, lysozyme activity can be detected in serum and synovial fluid samples from patients suffering from rheumatoid arthritis using a test system which is based on the lysis of the cell wall of *Micrococcus lysodeiktikus* (as agar diffusion test). In contrast thereto, the examples provided herein clearly show that lysozyme activity in healthy patients is not detectable or only to a very low degree. It has to be noted that Pruzanski W et al. did not determine lysozyme activity in synovial fluid of healthy individuals so that the lysozyme activity disclosed therein cannot be an indicator for determining whether an individual suffers from rheumatoid arthritis.

It was also surprisingly found that other infection markers known from wound infections, such as elastase, do not show an enzymatic activity in synovial fluid. This is a surprising finding because it can be expected that infections may provoke in a mammal body the secretion of the same or at least similar substances.

"Synovial joint", as used herein, refers to plane joints (or gliding joint), hinge joints, pivot joints, condyloid joints (or ellipsoidal joints), saddle joints and ball and socket joints. All of these joints are filled with synovial fluid, which is required to reduce friction between the articular cartilage of synovial joints during movement.

"Synovial joint infection", as used herein, refers to infections caused by bacteria, fungi and/or viruses. These infections may be the result of a mechanical injury causing the infiltration of bacteria, fungi and/or viruses into a synovial joint. However, joint infections may also occur in the course or after surgery. A further source of infection can be blood if the blood of the mammal contains respective microorganisms and/or viruses.

Common microorganims causing synovial joint infections are *Staphylococcus aureus, Staphylococcus epidermidis,* hemolysing streptococci of group A, pneumococci, *Pseudomonas aeruginosa, Proteus mirabilis, E. coli, Haemophilus influenzae,* propionibacteria and salmonellae. Furthermore, also other aerobic and anaerobic germs as well as fungi like *Candida albicans* may be responsible for synovial joint infections (e.g. Hepburn MJ, et al. Rheumatol Int. (2003) 23:255-7; Lequerre T, et al. (2002) Joint Bone Spine 69(5):499-501)

The term "substrate", in particular "lysozyme substrate", as used herein, includes compounds, which can be converted (e.g. hydrolysed, degraded) by enzymes like lysozyme or which interact in another specific way with enzymes.

According to a preferred embodiment of the present invention the synovial fluid sample is of a mammal showing symptoms of a synovial joint condition.

It is particularly preferred to detect synovial joint infections in mammals suspected to suffer from a synovial joint condition, since obtaining synovial fluid is invasive and requires punctuation of the joint.

In order to treat mammals showing symptoms of a synovial joint condition more efficiently by using the most appropriate medication it is important to know the cause of the joint condition. This can be achieved with the method of the present invention because it allows to discriminate between a synovial joint infection and a non-infectious inflammatory condition of a synovial joint. This discrimination is possible due to the fact that lysozyme activity can only be determined in synovial joints infected with microorganisms like bacteria and fungi as well as with viruses. Other conditions of synovial joints which are not caused by infections do not show lysozyme activity at all or to a low extent.

Therefore, another aspect of the present invention relates to a method for discriminating between a synovial joint infection and a non-infectious inflammatory condition of a synovial joint comprising the steps of
- contacting a synovial fluid sample of a mammal showing symptoms of a synovial joint condition with at least one lysozyme substrate,
- detecting a synovial joint infection when a conversion of said at least one substrate is determined in said sample and
- detecting a non-infectious inflammatory condition when substantially no lysozyme activity is determined.

"Substantially no lysozyme activity" and "no lysozyme activity", as used herein refers to a lysozyme activity which is not detectable by the method described herein, in particular in example 1, however preferably of less than 200 U/ml.

Lysozyme activity can be determined by using various methods known in the art. Preferred methods involve the conversion of a specific substrate into a product which can thereafter been detected and quantified. Such methods may involve also substrates which are able to release detectable substances like dyes. A particularly preferred method to determine lysozyme activity is described, for instance, in EP 2 433 138. The lysozyme substrate used in the methods described therein is preferably a peptidoglycan and/or chitosan, or oligomers of these, more preferably a peptidoglycan and/or chitosan dyed with a vinyl sulfone dye. The peptidoglycan may be derived from *Micrococcus lysodeictikus.*

The substrate concentration of stained peptidoglycan, for instance, can be within a range of 1 mg/ml to 50 mg/ml, preferably 10 mg/ml - 20 mg/ml, in particular 15 mg/ml.

The lysozyme substrate is incubated with the synovial fluid sample for a defined time or time range. It is particularly preferred to incubate the synovial fluid sample with the lysozyme substrate for 1 to 30 min, preferably 1 to 20 min, more preferably for 1 to 15 min, more preferably for 1 to 10 min, more preferably for 2 to 30 min, more preferably 2 to 20 min, more preferably for 2 to 15 min, more preferably for 2 to 10 min, more preferably for 5 to 30 min, more preferably 5 to 20 min, more preferably for 5 to 15 min, more preferably for 5 to 10 min.

The method of the present invention has the advantage that the synovial fluid used therein can be used without any preparation step. Thus, synovial fluid obtained from the joint of a mammal can be directly used to assess its lysozyme activity. This is surprising since the methods known in the art do not use synovial fluid directly to determine lysozyme activity. For instance, Pruzanski W et al. suggest to centrifuge the synovial fluid to be analysed and to dialyze it before contacting it with the lysozyme substrate.

According to a preferred embodiment of the present invention the non-infectious inflammatory condition of a synovial joint is inflammatory arthritis, preferably rheumatoid arthritis, acute aseptic arthritis or osteoarthritis, osteochondrosis and inflammation caused by traumatic events.

According to a further preferred embodiment of the present invention the synovial joint infection is a bacterial, fungal or viral infection, wherein bacterial infections may be preferably caused by *Staphylococcus aureus, Staphylococcus epidermidis,* hemolysing streptococci of group A, pneumococci, *Pseudomonas aeruginosa, Proteus mirabilis, E. coli, Haemophilus influenzae,* propionibacteria and salmonellae, and fungal infections may preferably caused by *Candida albicans.*

Synovial joint infections may be caused by one or more of the organisms listed above. Infections caused by one or more of these organisms can be treated with medicaments like antibiotics as well-known in the art.

The method of the present invention is preferably used with samples obtained from mammals which show symptoms of a synovial joint condition. The symptoms of a synovial joint condition include preferably pain, joint swelling, fever and/or inability to move the limb with the infected joint.

According to another preferred embodiment of the present invention the synovial joint infection is detected when the lysozyme activity within the sample is at least 500 U/ml, preferably at least 600 U/ml, more preferably at least 700 U/ml, more preferably at least 800 U/ml, more preferably at least 900 U/ml, more preferably at least 1,000 U/ml.

It turned out that the detection of a lysozyme activity within the synovial fluid of at least 500 U/ml indicates that said synovial fluid is derived from a joint having an infection caused by one of the pathogens/organisms mentioned above.

According to a preferred embodiment of the present invention a non-infectious inflammatory condition is detected when the lysozyme activity within the sample is less than 500 U/ml, preferably less than 400 U/ml, more preferably less than 300 U/ml, more preferably less than 200 U/ml.

According to a particularly preferred embodiment of the present invention the at least one lysozyme substrate is peptidoglycan or a derivative thereof.

Lysozyme is known to destroy bacterial cell walls by hydrolysing 1,4-beta-linkages between N-acetylmuramic acid and N-acetyl-D-glucosamine residues in peptidoglycan. In addition to its lytic activity against Gram positive bacteria, lysozyme can act as a non-specific innate opsonin by binding to the bacterial surface, reducing the negative charge and facilitating phagocytosis of the bacterium before opsonins from the acquired immune system arrive.

In another embodiment, provided herein the lysozyme substrate may have a structure A-I (Formula I) wherein, A is an anchor and I is an indicator region, wherein the indicator (I) or a motif therein is conjugated to the anchor and the conjugate is a substrate for lysozyme (see WO 2017/168249).

In some embodiments, the anchor region (A) is associated with the indicator region (I) via an enzyme recognition site (S). Under this embodiment, the enzyme recognition site is a structure or a motif that allows binding to an enzyme.

In one embodiment, the enzyme recognition site (S) is naturally present in the anchor region. In another embodiment, the enzyme recognition site (S) is introduced in the anchor region via chemical modification. Alternately, the enzyme recognition site (S) may be naturally present in the indicator region (I) or synthetically introduced in the indicator region (I) via one or more chemical modifications.

In one embodiment, the chemical entity of Formula I comprises an anchor (A) which is associated with the indicator (I), either covalently or non-covalently. Particularly, the association between the anchor region (A) and the indicator region (I) is mediated via a covalent interaction. As is understood in the art, covalent bonds involve sharing of electrons between the bonded atoms. In contrast, non-covalent bonds may include, for example, ionic interactions, electrostatic interactions, hydrogen bonding interactions, physiochemical interactions, van der Waal forces, Lewis-acid/Lewis-base interactions, or combinations thereof.

In one embodiment, the anchor A is associated with the indicator I via a covalent interaction to form the recognition site S. In another embodiment, the anchor A is associated with the indicator I via a covalent interaction that is not a part of the recognition site S.

In some embodiments, the chemical entity further comprises an enzyme-labile or enzyme-reactive region (R). In one embodiment, the reactive region (R) is a part of the anchor region. In another embodiment, the reactive region (R) is a part of the indicator region (I). Still further, the reactive region (R) is a part of the enzyme recognition site (S).

In one embodiment, the reactive region (R) interacts with lysozyme or myeloperoxidase, or a combination thereof.

The backbone of the lysozyme substrate can be chitosan, peptidoglycan or any derivative thereof, whereas peptidoglycan is preferred.

The reactive dyes for staining the substrate may have different amounts of sulfonate groups ranging from 1-6 sulfonate groups.

The backbone can be stained with Reactive Red 120 or reactive green 19 (6 sulfonate groups); with Reactive Blue 109 (5 sulfonate groups); with Reactive Brown 10 (4 sulfonate groups).; with Reactive blue 5 (3 sulfonate group); with Reactive Black 5 or reactive violet 5 (2 sulfonate groups); with Reactive Orange 16 or Reactive blue 19 (1 sulfonate group). Preferred are dyes containing 1-2 sulfonate groups such as reactive black 5 or reactive blue 19.

According to a preferred embodiment of the present invention the synovial fluid sample is further contacted with at least one myeloperoxidase substrate and a synovial joint infection is detected when a conversion of said at least one substrate is determined.

Myeloperoxidase (MPO) activity is known to be significantly increased in infectious tissue. The additional determination/quantification of MPO activity in a synovial fluid sample may be used to confirm that the synovial joint is infected.

According to another preferred embodiment of the present invention the synovial joint infection is detected when the myeloperoxidase activity within the sample is at least 0.1 U/ml, preferably at least 0.2 U/ml, more preferably at least 0.3 U/ml, more preferably 0.4 U/ml, more preferably at least 0.5 U/ml, more preferably at least 1 U/ml.

According to a preferred embodiment of the present invention the at least one myeloperoxidase substrate is selected from the group consisting of Guajacol, 3,4-Diamino benzoic acid, 4-Amino-3-hydroxy benzoic acid, Methyl-3,4-diaminibenzoate, 3-Amino-4-hydroxy benzoic acid, 2,3-Diamino benzoic acid, 3,4-Dihydroxy benzoic acid, 2-Amino phenol, 2-Amino-3-methoxy benzoic acid, 2-Amino-4-methylphenol, 3,3',5,5'-tetramethylbenzidin, 2,2'-azino-bis(3-ethylbenzthiazoline-6-sulphonic acid), crystal violet, leuko crystal violet and sinaptic acid Fast Blue RR modified with isocyanate.

According to the present invention MPO activity can be determined using the following detection system for the detection of elevated MPO levels in synovial fluid. The system comprises of a reaction vessel filled with a defined volume of a phosphate buffer (100-1400 µl, sodium or potassium, pH 6.2-8.0, 10.0-100 mM). The synovial fluid sample (100-1400µl) is then transferred into the reaction vessel containing the pre added buffer. Therefore, the dilution factor can range from 10:1 to 1:10. The most preferred dilution factor ranges from 2:1 - 1:2.

The MPO substrate is added in solid form, for instance as tablet, and can contain one of the aforementioned substrates and most preferred a substrate selected from the group consisting of Guajacol, 3,4-Diamino benzoic acid, 4-Amino-3-hydroxy benzoic acid, Methyl-3,4-di-aminibenzoate, 3-Amino-4-hydroxy benzoic acid, 2,3-Diamino benzoic acid, 3,4-Dihydroxy benzoic acid, 2-Amino phenol, 2-Amino-3-methoxy benzoic acid and 2-Amino-4-methylphenol.

If a tablet is used the tablet may have a weight of 10 - 100 mg where the optimum is 20 mg. The tablets may be pressed with a pressure of 2 t for 10 seconds with a diameter of 5 mm.

According to a preferred embodiment of the present invention a tablet may have the following composition: 0,01-0,10 mg Na₂CO₃*1,5 H₂O₂, ideally 0,03-0,05 mg 0,01-1 mg active compound, ideally 0,20-0,40 mg Maltose can be used as filler and may be added to gain the desired final weight

After the addition of the substrate to the synovial fluid sample, the whole system is well mixed and the enzymatic reaction starts immediately. Depending on the level of MPO activity a visible colour change of the solution can be observed. The arising colour is depending on the active ingredient and can be yellow, orange, red, brown, black or any combination thereof.

According to another preferred embodiment of the present invention the mammal is selected from the group consisting of human, cattle, pigs, sheep and horses.

The present invention is further illustrated by the following examples, however, without being restricted thereto.

### EXAMPLES

### Example 1: Determination of lysozyme activity in synovial fluid samples obtained from healthy patients and patients suffering from rheumatoid arthritis and microbial infections of a synovial joint

Lysozyme is an enzyme, which is produced and secreted by cells of the innate immune system. Peptidoglycan is the main building block of cell walls from gram positive bacteria and can be cleaved by lysozyme. This is a natural defence mechanism from the immune system to cope with bacteria by digesting their cell walls. It was demonstrated, that in 100% (see Table 1, Sensitivity) of the tested synovia samples which were diagnosed as septic by the responsible clinicians, the lysozyme activity was elevated. Moreover, 100 % (see Table 1, Specificity) of the samples which were classified as negative or inflammatory did not show elevate lysozyme levels.

The activities of lysozyme were measured as follows: 290 µl of a 0.45 mg/ml peptidoglycan solution (from *Micrococcus luteus*) in a sodium phosphate buffer (50 mM, pH 6.5) were incubated with 10µl sample (synovial fluid). Absorbance measurements were carried out at 450 nm for 10 minutes at 37°C.

**Table 1: Sensitivity, specificity, positive and negative prediction values (PPV, NPV) for the detection of infection in equine synovia samples.**

| | Value | % |
|---|---|---|
| Sensitivity | 1 | 100 |
| Specifcity | 1 | 100 |

### Example 2: Activity measurement of lysozyme with dyed peptidoglycan

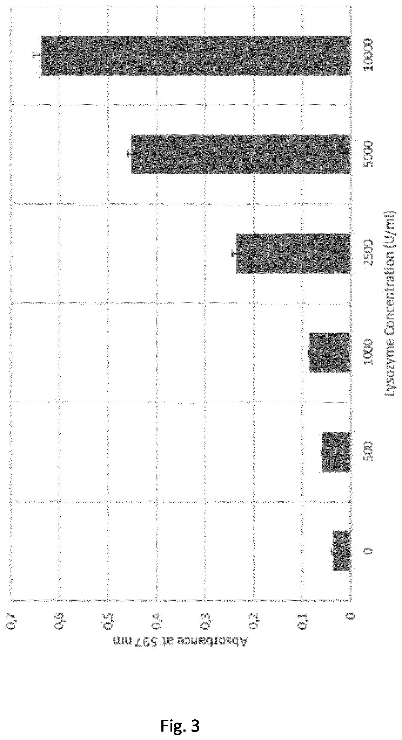

7.5 mg peptidoglycan-reactive black 5 were suspended in 0.990 ml 0.9 % NaCl solution. 10 µl of different lysozyme concentrations were added to the reaction solution to final concentrations ranging from 0 U/ml - 10000 U/ml (every concentration in triplicates). All samples were incubated at 37 °C for 60 minutes and shaking at 1400 rpm. After the incubation step all samples were centrifuged at 10000 g for 5 minutes. 100 µl of each supernatant were transferred into a 96 well plate and absorbances were measured at 597 nm. Absorbance values are shown in Fig. 3. All lysozyme concentrations could be clearly distinguished.

### Example 3: Dye release over 60 minutes for 10000 U/ml lysozyme

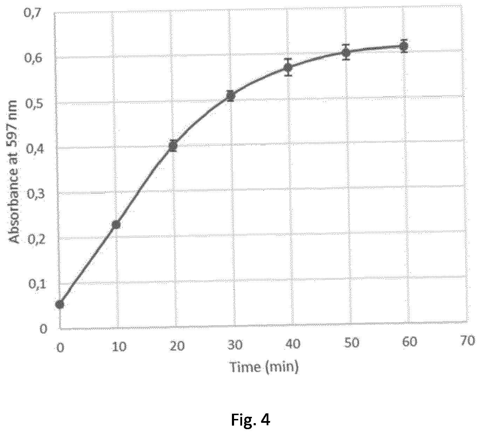

7.5 mg/ml peptidoglycan-reactive black 5 were suspended in 0.990 ml 0.9 % NaCl solution. 10 µl of a lysozyme solution were added to a final concentration of 10000 U/ml. All samples were incubated at 37 °C for 10 - 60 minutes and 1400 rpm. Every 10 minutes triplicates were centrifuged (10000g, 5 min) and 100 µl of the supernatants were transferred into a 96 well plate. The absorbance was measured at 597 nm. The time-depending dye release is shown in Fig. 4. Already after 10 minutes the

### Example 4: Optimisation of the substrate concentration using peptidoglycan-reactive black 5

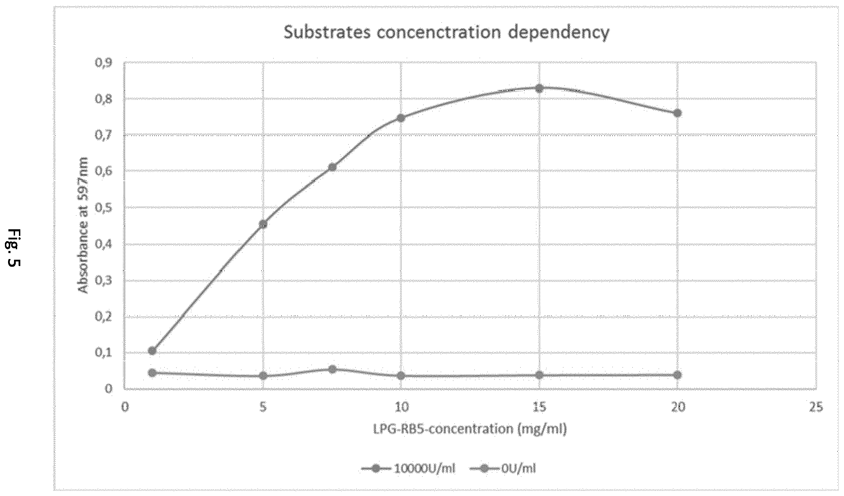

Different amounts of substrate were suspended in 0.9 % NaCl solution (5 mg/ml - 20 mg/ml, in triplicates). 10 µl of a lysozyme solution were added to all different substrate concentrations to a final lysozyme concentration of 10000 U/ml. 10 µl of H₂O served as negative controls for all different substrate concentrations (all in triplicates). All samples were incubated at 37 °C for 60 minutes and 1400 rpm. After the incubation all samples were centrifuged (10000 g, 5 min) and 100µl of the supernatants were transferred into a 96 well plate. Absorbances were measured at 597 nm. The substrate depending dye release can be seen in Fig. 5.

### Example 5: Determination of MPO activity in synovial fluid samples obtained from healthy patients and patients suffering from rheumatoid arthritis and microbial infections of a synovial joint

MPO is also an enzyme from the innate immune system and acts as a natural defence mechanism against bacteria by producing highly active oxygen species. One possible MPO reaction is the oxidation of substrates using H₂O₂ as cofactor. The second mechanism known is the chlorination activity which can also lead to the oxidation and/or cleavage of molecules.

Many chromogenic substrates are known to change colour upon oxidation reactions and can therefore be used for the detection of MPO activity.

A guajacol based assays was used for the determination of MPO actiovity and was carried out as follows:
10 µl of a synovial fluid sample were added to 290 µl of reaction solution containing 187.4 mM guajacol in a potassium phosphate buffer (100 mM, pH 7.0) and 0.35 mM H₂O₂. The absorbance change at 470 nm was recorded over a time period of 60 seconds.

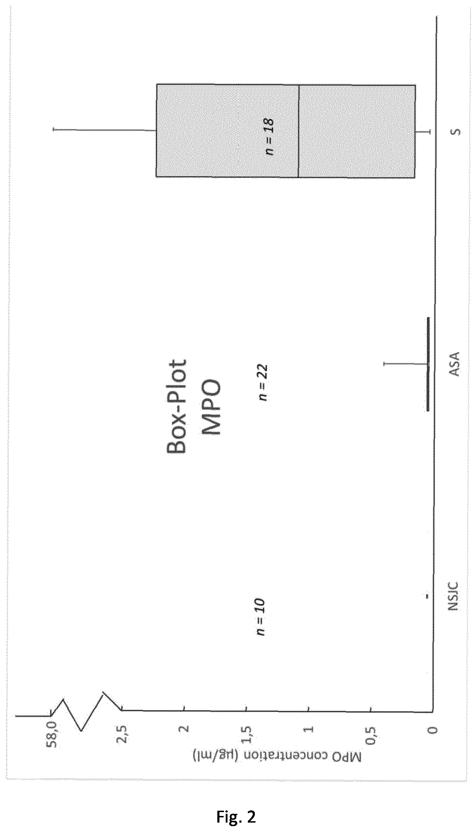

On the one hand we could show that MPO activity was elevated in 75% of all investigated synovia samples which were classified as septic (sensitivity see table 2). On the other hand, we could demonstrate that the MPO activity was low for 90% of the negative classified samples (aseptic). This leads to a sensitivity of 75.0 % and a specificity of 90.0 % (see table 2 and Fig. 2).

**Table 2: Sensitivity, specificity, PPV und NPV for MPO as marker for the detection of infection in synovia**

| | | % |
|---|---|---|
| Sensitivity | 0,75 | 75,0 |
| Specifity | 0,9 | 90 |
| PPV | 0,82 | |
| NPV | 0,86 | |

### Example 6: Determination of elastase activity in synovial fluid samples obtained from healthy patients and patients suffering from rheumatoid arthritis and microbial infections of a synovial joint

The enzyme elastase is also described as a marker enzyme in synovia. Elastase is a proteolytic enzyme and cleaves proteins with specific recognition sequence. This can be used to cleave of chromophores from substrate proteins. The following assay was used for the determination of the elastase activity.

Methoxysuccinyl-Ala-Ala-Pro-Val-p-nitroanilid was used as substrate. By cleaving the p-Nitroaniline from the peptide, a yellow colour is emerging and can be measured at 405 nm. The substrate was dissolved in Dimethyl sulfoxide and dilute with a sodium phosphate buffer (10-50 mM, pH 6.0 - 8.0). The final substrate concentration was 1-5 mM.

Elastase activity was not detectable in any synovia sample which is a surprisingly different result compared to the fact that elastase activity could be detected in infected wound fluid. This is particularly surprising considering that it is known from literature, that Elastase is detectable in synovial fluid by ELISA assays.

In comparison to the system for the detection of wound infection a positive control is not necessary as the samples are taken by puncture and therefore, wrong or insufficient sampling is avoided.

### Example 7: Determination of cathepsin activity in synovial fluid samples obtained from healthy subjects and subjects suffering from rheumatoid arthritis and microbial infections of a synovial joint

10 µl synovial fluid (equine) of healthy horses, subjects suffering from rheumatoid arthritis and micro-bial infections were added each to 90 µl of a 3 mM solution of Cathepsin G substrate (N-Succinyl-Ala-Ala-Pro-Phe p-nitroanilide) (stock solved 30 mM in DMSO) in sodium phosphate buffer (25 mM, pH 7.0, 0.5 M NaCl). Absorbances were measured at 405 nm for 20 minutes at 37 °C. 9 samples were analysed: 3 septic and 3 aseptic (arthritis) synovia samples as well as 3 synovial fluid samples from healthy subjects. Cathepsin G activity could not be measured in any sample.

## Claims

1. A method for detecting a synovial joint infection comprising the steps of
- contacting a synovial fluid sample with at least one lysozyme substrate, and
- detecting a synovial joint infection when a conversion of said at least one substrate is determined.

2. Method according to claim 1, wherein the synovial fluid sample is of a mammal showing symptoms of a synovial joint condition.

3. Method for discriminating between a synovial joint infection and a non-infectious inflammatory condition of a synovial joint comprising the steps of
- contacting a synovial fluid sample of a mammal showing symptoms of a synovial joint condition with at least one lysozyme substrate,
- detecting a synovial joint infection when a conversion of said at least one substrate is determined in said sample and
- detecting a non-infectious inflammatory condition when substantially no lysozyme activity is determined.

4. Method according to claim 3, wherein the non-infectious inflammatory condition is inflammatory arthritis, preferably rheumatoid arthritis, acute aseptic arthritis or osteoarthritis, osteochondrosis and inflammation caused by traumatic events.

5. Method according to any one of claims 1 to 4, wherein the synovial joint infection is a bacterial , fungal or viral infection.

6. Method according to any one of claims 2 to 5, wherein symptoms of a synovial joint condition include pain, joint swelling, fever and/or inability to move the limb with the infected joint.

7. Method according to any one of claims 1 to 6, wherein the synovial joint infection is detected when the lysozyme activity within the sample is at least 500 U/ml, preferably at least 700 U/ml, more preferably at least 1,000 U/ml.

8. Method according to any one of claims 1 to 7, wherein a non-infectious inflammatory condition is detected when the lysozyme activity within the sample is less than 500 U/ml.

9. Method according to any one of claims 1 to 8, wherein the at least one lysozyme substrate is peptidoglycan or a derivative thereof.

10. Method according to any one of claims 1 to 9, wherein the synovial fluid sample is further contacted with at least one myeloperoxidase substrate and a synovial joint infection is detected when a conversion of said at least one substrate is determined.

11. Method according to claim 10, wherein the synovial joint infection is detected when the myeloperoxidase activity within the sample is at least 0.4 U/ml.

12. Method according to claim 10 or 11, wherein the at least one myeloperoxidase substrate is selected from the group consisting of Guajacol, 3,3',5,5'-tetramethylbenzidin, 2,2'-azino-bis (3-ethylbenzthiazoline-6-sulphonic acid), crystal violet, leuko crystal violet, sinaptic acid Fast Blue RR modified with Isocyanat, 3,4-Diamino benzoic acid, 4-Amino-3-hydroxy benzoic acid, Methyl-3,4-diaminibenzoate, 3-Amino-4-hydroxy benzoic acid, 2,3-Diamino benzoic acid, 3,4-Dihydroxy benzoic acid, 2-Amino phenol, 2-Amino-3-methoxy benzoic acid and 2-Amino-4-methylphenol.

13. Method according to any of claims 1 to 12, wherein the mammal is selected from the group consisting of human, cattle, pigs, sheep and horses.
